(19) Europäisches Patentamt
European Patent Office
Office européen des brevets

(11) Numéro de publication: **0 067 106**
**B1**

(12) # FASCICULE DE BREVET EUROPEEN

(45) Date de publication du fascicule du brevet: **10.04.85**

(51) Int. Cl.⁴: **C 07 C 93/06,** A 61 K 31/13 // C07D303/22, C07C49/733

(21) Numéro de dépôt: **82400998.9**

(22) Date de dépôt: **01.06.82**

(54) **Nouveaux éthers de phénol actifs sur le système cardiovasculaire, leur procédé de préparation et leur utilisation dans des médicaments.**

(30) Priorité: **05.06.81 FR 8111242**

(43) Date de publication de la demande: **15.12.82 Bulletin 82/50**

(45) Mention de la délivrance du brevet: **10.04.85 Bulletin 85/15**

(84) Etats contractants désignés: **AT BE CH DE FR GB IT LI LU NL SE**

(56) Documents cités:
**FR - A - 2 018 626**
**FR - A - 2 196 799**
**FR - A - 2 384 740**

**Le dossier contient des informations techniques présentées postérieurement au dépôt de la demande et ne figurant pas dans le présent fascicule.**

(73) Titulaire: **SANOFI, Société dite:, 40, Avenue George V, F-75008 Paris (FR)**

(72) Inventeur: **Demarne, Henri, Le Florence avenue Major Flandre, F-34000 Montpellier (FR)**
Inventeur: **Wagnon, Jean, 195 rue Michel Ange, F-34000 Montpellier (FR)**

(74) Mandataire: **Gillard, Marie-Louise et al, Cabinet Beau de Loménie 55, Rue d'Amsterdam, F-75008 Paris (FR)**

**EP 0 067 106 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne en tant que produits industriels nouveaux des substances chimiques dérivées d'éthers de phénols ainsi que leurs sels d'addition d'acides et les isomères optiques desdits dérivés.

L'invention concerne également un procédé de préparation des nouveaux composés ainsi que leur application en thérapeutique.

Les composés selon l'invention sont choisis parmi l'ensemble constitué par:

a) les racémiques et les isomères optiques répondant à la formule générale:

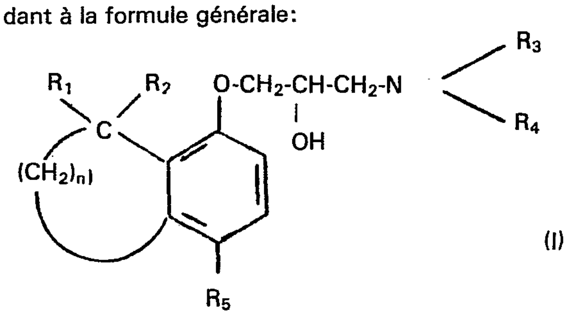

(I)

dans laquelle:

— $R_1$ et $R_2$ pris ensemble représentent un atome d'oxygène = O ou encore $R_1$ représente l'hydrogène et $R_2$ représente un radical OH,

— $R_3$ désigne un atome d'hydrogène ou un radical alkyle droit ou ramifié ayant de 1 à 6 atomes de carbone,

— $R_4$ représente un radical alkyle droit ou ramifié ayant de 1 à 6 atomes de carbone, ou un groupe alkynyle ayant 2 à 6 atomes de carbone,

— $R_5$ désigne un atome d'hydrogène, un groupe alkyle ayant de 1 à 4 atomes de carbone ou un groupe acylamino,

— n = 3 ou 4;

b) leurs sels d'addition d'acides.

Les composés (I) où $R_1$ et $R_2$ représentent un atome d'oxygène sont obtenus en deux étapes à partir des cétones ortho hydroxylés 1, selon le schéma réactionnel:

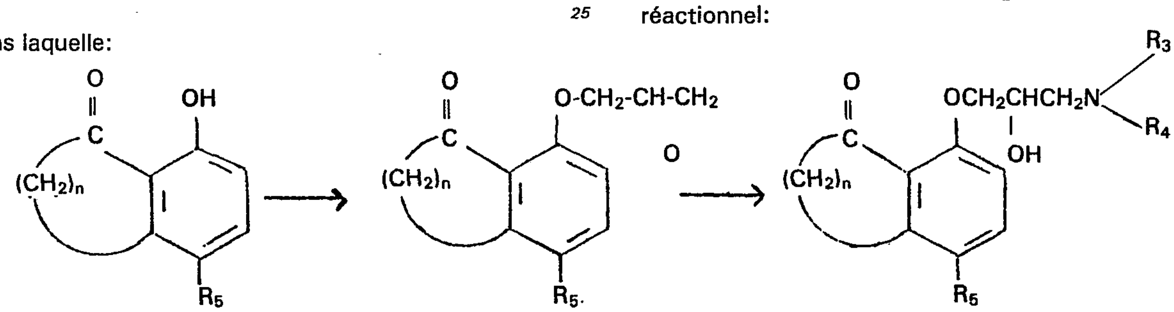

1 2 I ($R_1$, $R_2$ = O)

Lorsque n = 3, les cétones 1 sont connues et décrites dans la littérature chimique, par exemple dans The Journal of Organic Chemistry 28, 325 (1963) et dans The Journal of Chemical Society 1964, 2816.

Quand n = 4, la cétone 1 peut être obtenue à partir de l'orthobromoanisole et la cyclopentanone par la suite de réactions suivantes:

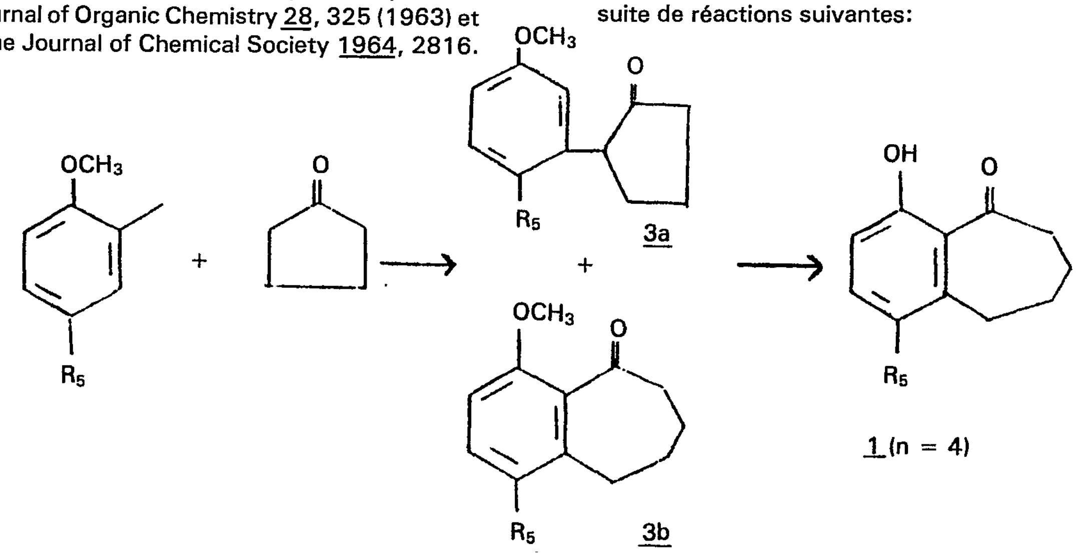

3a 3b 1 (n = 4)

L'obtention du mélange de cétones 3 avait été décrite dans Tetrahedron 21, 95 (1972), mais la séparation des constituants est malaisée.

Il a été trouvé que, par action d'un agent de déméthylation, tel que le tribromure de bore sur le mélange, des deux cétones, seul le composé 3b est déméthylé alors que 3a est décomposé. Il est ensuite facile de séparer les deux constituants du mélange et d'obtenir le produit 1 (n = 4) avec un rendement d'environ 50%.

A partir des cétones 1, l'action de l'épichlorhydrine en présence d'un agent alcalin, tel que la soude au sein d'un solvant comme l'eau, l'éthanol ou un polyalcool tel que le diéthylèneglycol ou encore un éther de polyol tel que le méthoxy-2 éthanol, à une température comprise entre 50 et 120°C, conduit aux époxydes 2. L'ouverture de ces époxydes 2 par une amine $\begin{matrix}R_3\\R_4\end{matrix}\!>NH$ au sein d'un solvant comme un alcool aliphatique inférieur tel que l'éthanol, à une température comprise entre 50 et 120°C conduit aux composés (I). Par réduction ultérieure, par exemple par du borohydrure de sodium dans un alcool aliphatique inférieur, on obtient les composés (I) dans lesquels $R_1$ = H et $R_2$ = OH.

Dans tous les cas, par action sur les composés (I) d'un acide minéral ou organique au sein d'un solvant approprié, on obtient les sels correspondants.

La séparation des isomères optiques présents dans les produits (I) peut, si nécessaire, être effectuée selon une méthode connue, par le traitement du produit racémique avec un acide optiquement actif, tel que l'un des acides D et L tartriques, D et L dibenzoyltartriques ou D et L di-p-toluoyltartriques.

Les exemples suivants illustrent l'invention.

*Exemple 1*

*(Oxo-8 tétrahydro-5,6,7,8 naphthyl-1 oxy)-1 hydroxy-2 tertiobutylamino-3 propane*

a) *(Oxo-8 tétrahydro-5,6,7,8 naphthyl-1 oxy)-1 époxy-2,3 propane*

A la solution chauffée à 80°C de 3,2 g d'hydroxy-8 tétralone-1 et 20 ml d'épichlorhydrine dans 20 ml de méthoxy-2 éthanol, on ajoute 1,8 g de soude et on chauffe au reflux pendant 1 h. On ajoute de l'eau et on extrait avec du chlorure de méthylène. On sépare la phase organique, on sèche et on évapore le solvant.

Le résidu est chromatographié sur colonne de silice et, par élution au chlorure de méthylène, on obtient 3 g du produit attendu utilisé tel quel pour l'étape suivante.

b) *CM 7285*

On chauffe au reflux pendant 3 h le mélange de 3 g de l'époxyde obtenu ci-dessus et 2,5 g de tertiobutylamine dans 50 ml d'éthanol absolu. On évapore le solvant et on reprend le résidu dans l'acide chlorhydrique dilué. On extrait la phase aqueuse avec de l'éther puis on l'alcalinise jusqu'à pH 9. On extrait avec de l'éther, on sèche la solution et on évapore le solvant.

Le résidu solide est recristallisé dans l'éther isopropylique et fournit des cristaux crème (1,6 g); Fc: 76-80°C.

*Exemple 2*

*(Tertiobutylamino-3 hydroxy-2 propoxy)-4 tétrahydro-6,7,8,9 5H-benzocycloheptènone-5*

(n° de code CM 7630; I $R_1R_2$ = O, $R_3$ = H, $R_4$ = $C(CH_3)_3$, n = 4, $R_5$ = H)

a) *Hydroxy-4 tétrahydro-6,7,8,9 5H-benzocycloheptènone-5* (1 n = 4)

On prépare selon P. Caubert et col., Tetrahedron 21, 95, 1972, à partir d'orthobromoanisole et de cyclopentanone, un mélange des cétones 3a et 3b; E./1,2 mmHg: 124-130°C.

A une solution de 4,2 g du mélange précédent dans 50 ml de chlorure de méthylène refroidie à 0°C, on ajoute en 10 min la solution de 11 g de tribromure de bore dans le chlorure de méthylène puis on laisse au repos pendant 40 h à température ambiante. On verse la solution dans l'eau et on neutralise par addition de bicarbonate de sodium. On extrait la phase aqueuse avec du chlorure de méthylène et on filtre sur colonne de silice. Par élution par un mélange éther de pétrole-éther 8:2 vol/vol, on obtient le phénol attendu (2,15 g) sous forme d'une huile caractérisée par son spectre IR et son spectre RMN.

b) *(Epoxy-2,3 propoxy)-4-tétrahydro-6,7,8,9 5H-benzocycloheptènone-5* (2 n = 4)

On chauffe au reflux pendant 4 h le mélange de 2 g de phénol obtenu ci-dessus, 25 ml d'épichlorhydrine et 1,2 g de soude dans 25 ml de méthoxy-2 éthanol. On ajoute de l'eau puis on extrait avec de l'éther.

On lave la solution avec de l'eau, on sèche sur sulfate de sodium et on évapore le solvant à siccité.

Le résidu est utilisé tel quel pour l'étape suivante.

c) *CM 7630*

Le résidu de l'étape précédente (2,5 g) est dissous dans 35 ml d'éthanol absolu. On ajoute 1,5 g de tertiobutylamine et on porte au reflux durant 4 h. On évapore le solvant à siccité et on reprend le résidu dans l'acide chlorhydrique dilué. On extrait la phase aqueuse avec de l'éther puis on l'alcalinise par le carbonate de sodium. Il se sépare un solide qu'on recristallise dans l'éther isopropylique.

On obtient des cristaux incolores (1,7 g); Fc: 74,5-76°C.

*Exemple 3*

*(Hydroxy-8 tétrahydro-5,6,7,8 naphthyl-1 oxy)-1 hydroxy-2 tertiobutylamino-3 propane, fumarate acide*

n° de code CM 7633; I $R_1$ = H, $R_2$ = OH, $R_3$ = H, $R_4$ = $C(CH_3)_3$ (n = 3), $R_5$ = H

On dissout 1,5 g du composé de l'exemple 1 dans 20 ml de méthanol et on refroidit à 0°C puis on ajoute la solution de 1,5 g de borohydrure de sodium dans 20 ml d'eau et on agite pendant 2 h à 0°C.

On détruit l'excès de borohydrure par addition d'une petite quantité d'acétate d'éthyle, puis on dilue le mélange avec de l'eau et on évapore le méthanol. On extrait la phase aqueuse avec de l'éther puis on extrait la solution éthérée avec une solution diluée d'acide chlorhydrique. La solution chlorhydrique est alcalinisée par le carbonate de sodium. On extrait avec de l'éther, on sèche sur sulfate de sodium et on évapore le solvant à siccité.

Il reste une huile que l'on transforme en fumarate acide par addition d'une quantité stoechiométrique d'acide fumarique dissoute dans le minimum d'éthanol.

On essore les cristaux et on recristallise dans le mélange éthanol absolu-éther isopropylique.

Finalement, on obtient des cristaux (1,7 g); Fc: 161-164°C.

*Exemples 4 à 13*

En opérant comme dans les exemples 1 et 2, mais en faisant varier l'amine $\frac{R_3}{R_3}>NH$ et/ou le produit de départ 1, on obtient les produits dont les caractéristiques sont rassemblées dans le tableau I suivant.

*Exemples 14 à 17*

En opérant comme dans l'exemple 3, mais en faisant varier le composé cétonique de départ, on obtient les produits mentionnés dans le tableau II suivant.

Tableau I

| Exemple n° | N° de code | $R_1, R_2$ | $R_5$ | n | $-N<^{R_3}_{R_4}$ | Sel ou base (solvant de recristallisation) | F. (°C) |
|---|---|---|---|---|---|---|---|
| 4 | 7331 | = O | H | 3 | $-NH-CH(CH_3)_2$ | Base (chlorure de méthylène-éther isopropylique) | 65 |
| 5 | 7632 | = O | H | 3 | $CH_3$<br>\|<br>$-N-C-C_2H_5$<br>\| \|<br>$H\ CH_3$ | Fumarate acide (éthanol-acétate d'éthyle) | 136-138 |
| 6 | 7692 | = O | H | 4 | » » | Base (hexane-éther isopropylique) | 52-53 |
| 7 | 7700 | = O | $-NH-COCH_3$ | 3 | $-NH-CH(CH_3)_2$ | Base (méthanol-éther isopropylique) | 175-177 |
| 8 | 7706 | = O | $-CH_3$ | 3 | $-NH-C(CH_3)_3$ | Fumarate acide (éthanol-acétate d'éthyle) | 234-235 |
| 9 | 7709 | = O | H | 4 | $-NH-CH(CH_3)_2$ | Fumarate acide (acétone) | 174-178 |
| 10 | 7713 | = O | $-CH_3$ | 3 | $-NHCH_2-CH(CH_3)_2$ | Base (hexane) | 93-94 |
| 11 | 7760 | = O | $-CH_3$ | 3 | $CH_3$<br>\|<br>$-NH-C-C\equiv CH$<br>\|<br>$CH_3$ | Fumarate acide (acétate d'éthyle, éthanol) | 142 |
| 12 | 7994 | = O | H | 4 | $CH_3$<br>\|<br>$-NH-C-C\equiv CH$<br>\|<br>$CH_3$ | Fumarate acide (méthanol, acétone, acétate d'éthyle) | 135-137 |
| 13 | 7995 | = O | H | 4 | $NH-CH_2CH(CH_3)_2$ | Fumarate acide (méthanol, acétate d'éthyle) | 157-159 |

Tableau II

| Exemple n° | N° de code | $R_1, R_2$ | $R_5$ | n | $-N<^{R_3}_{R_4}$ | Sel ou base (solvant de recristallisation) | F. (°C) |
|---|---|---|---|---|---|---|---|
| 14 | 7693 | H, OH | H | 4 | $-NH-C(CH_3)_3$ | Base (1er isomère) (éther isopropylique, hexane) | 142-144 |
| 15 | 7694 | » | » | » | » | Base (2e isomère) (hexane-éther isopropylique) | 108-110 |
| 16 | 7707 | H, OH | $-CH_3$ | 3 | $-NH-C(CH_3)_3$ | Fumarate acide (acétate d'éthyle, éthanol) | 161-162 |
| 17 | 7800 | H, OH | $-CH_3$ | 3 | $CH_3$<br>\|<br>$-NH-C-C\equiv H$<br>\|<br>$CH_3$ | Fumarate acide (éthanol) | 153 |

Les produits de l'invention ont été étudiés en vue de déterminer leur activité pharmacologique et, plus spécialement, leur activité sur le système cardiovasculaire.

Les produits de l'invention ont été soumis aux épreuves pharmacodynamiques relatées ci-après.

*Action pharmacologique chez le chien*

Le chien est anesthésié au pentobarbital sodique administré par voie intraveineuse à la dose de 30 mg/kg. Une canule placée dans la veine saphène permet les injections intraveineuses des produits. L'animal est intubé et laissé en respiration spontanée.

La fréquence cardiaque et la pression artérielle systémique sont étudiées et les variations de ces paramètres sont observées après injection intraveineuse du produit à tester, chaque produit étant injecté à doses croissantes.

*Antagonisme des effets de l'isoprénaline*

L'antagonisme des produits vis-à-vis des effets cardiovasculaires $\beta$ stimulants de l'isoprénaline sur les récepteurs $\beta$ adrénergiques a été recherché. Les résultats sont présentés dans le tableau ci-dessous et exprimés en DI 50: il s'agit de la dose exprimée en mg/kg qui provoque l'inhibition de 50% de la tachycardie ($\beta_1$) et de l'hypertension ($\beta_2$) induite par l'isoprénaline administrée par voie intraveineuse.

*Antagonisme des effets de la noradrénaline*

L'antagonisme des produits vis-à-vis des effets vasculaires provoqués par l'administration intraveineuse de noradrénaline sur les récepteurs $\alpha$ adrénergiques a été recherché. Les résultats présentés dans le tableau ci-dessous sont exprimés en DI 50, c'est la dose (mg/kg) qui provoque l'inhibition de 50% de la réponse pressive due à l'administration intraveineuse de noradrénaline.

| N° code produits | DI 50 isoprénaline $\beta_1$ | $\beta_2$ | DI 50 noradrénaline mg/kg (réponse pressive) |
|---|---|---|---|
| 7285 | 0,03 à 0,1 | 0,03 | 0,3 à 3 |
| 7630 | 0,01 à 0,03 | 0,01 à 0,03 | 0,03 à 0,1 |
| 7632 | 0,01 à 0,1 | 0,01 à 0,1 | 0,03 à > 0,1 |
| 7694 | 0,03 à 0,1 | 0,01 à 0,03 | 0,1 à 0,3 |
| 7706 | 0,1 | 0,01 | 0,03 à 0,3 |
| 7760 | 0,1 | 0,03 | 0,1 à 0,3 |

Il ressort de ces résultats que les produits selon l'invention sont particulièrement actifs en tant qu'antagonistes des effets $\beta$ stimulants de l'isoprénaline sur les récepteurs $\beta$ adrénergiques et en tant qu'antagonistes des effets $\alpha$ stimulants de la noradrénaline sur les récepteurs $\alpha$ adrénergiques.

Certains produits provoquent une bradycardie et/ou provoquent une baisse de la pression artérielle systémique chez l'animal anesthésié.

Par ailleurs, ces produits sont peu toxiques. Ils peuvent donc être utilisés dans les indications thérapeutiques suivantes:

— traitement des troubles pathologiques en rapport avec une hyperproduction de catécholamines: tachycardies, palpitations, extrasystoles, hypertension;

— traitement de fond de la maladie angineuse, des séquelles d'infarctus, des troubles du rythme auriculaire et ventriculaire;

— traitement de fond de la maladie hypertensive;

— traitement de différents troubles neurologiques: états d'anxiété isolés ou avec localisation organique, cures de désintoxication, etc.

Ils peuvent être présentés sous les différentes formes d'administration orale, telle que comprimés dosés de 1 à 10 mg, d'administration rectale telle que suppositoires dosés de 1 à 10 mg et préparations injectables contenant de 0,5 à 5 mg de principe actif.

La posologie usuelle est de 1 à 2 comprimés à 5 mg par jour, mais, exceptionnellement, sous surveillance médicale, elle pourra dépasser notablement ce chiffre.

On donne ci-après quelques exemples de préparation galénique.

*Comprimés*

| | |
|---|---|
| CM 7630 | 5 mg |
| Cellulose microcristalline | 160 mg |
| Lactose | 187 mg |
| Stéarate de magnésium | 8 mg |
| | 360 mg |

*Suppositoires*

| | |
|---|---|
| CM 7630 | 10 mg |
| Suppocire C (mélange injectable d'esters d'acides gras naturels)<br>Labrafil 2130 C (huile de palme hydrogénée interestérifiée) | qsp 3 g |

**Revendications pour les Etats contractants: BE, CH, DE, FR, GB, IT, LI, LU, NL, SE**

1. Nouveaux produits, caractérisés en ce qu'ils répondent à la formule générale:

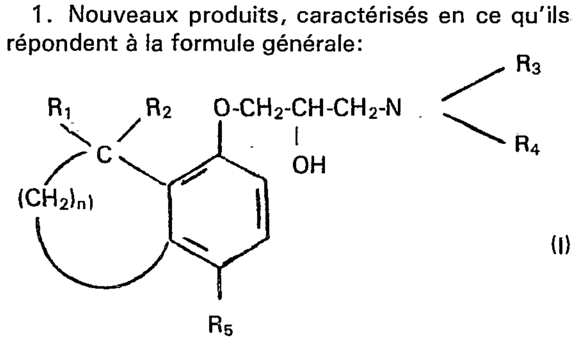

(I)

dans laquelle:

— $R_1$ et $R_2$ pris ensemble représentent un atome d'oxygène = O, ou encore $R_1$ représente l'hydrogène et $R_2$ représente un radical OH,
— $R_3$ désigne un atome d'hydrogène ou un radical alkyle droit ou ramifié ayant de 1 à 6 atomes de carbone,
— $R_4$ représente un radical alkyle droit ou ramifié ayant de 1 à 6 atomes de carbone ou un groupe alkynyle ayant de 2 à 6 atomes de C,
— $R_5$ désigne un atome d'hydrogène, un groupe alkyle ayant de 1 à 4 atomes de carbone ou un groupe acylamine,
— n = 3 ou 4,
ainsi que les sels d'addition d'acides desdits produits.

2. Nouveaux produits selon la revendication 1, caractérisés en ce qu'ils sont sous forme racémique ou sous forme de l'un de leurs isomères optiques.

3. Procédé de préparation des produits selon la revendication 1, caractérisé en ce que l'on fait réagir sur une cétone de formule:

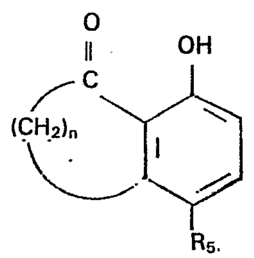

de l'épichlorhydrine en présence d'un agent alcalin au sein d'un solvant tel que de l'eau, de l'éthanol, un polyalcool ou un éther de polyol et que l'on fait réagir le produit obtenu sur une amine de formule $HN \langle {R_3 \atop R_4}$, après quoi on obtient les dérivés dans lesquels $R_1$ est H et $R_2$ est OH par réduction à l'aide d'un borohydrure de sodium.

4. Procédé selon la revendication 3, caractérisé en ce que les dérivés optiquement actifs sont obtenus par traitement du racémique correspondant à l'aide d'un acide optiquement actif.

5. Médicament actif notamment sur le système cardiovasculaire, caractérisé en ce qu'il contient, comme substance active, un produit selon l'une des revendications 1 et 2.

6. Médicament selon la revendication 5, caractérisé en ce qu'il est conditionné pour être administré sous forme orale, sous forme de suppositoires ou sous forme injectable à raison de 1 à 10 mg de principe actif par jour.

**Revendications pour l'Etat contractant:**
AT

1. Procédé pour l'obtention de nouveaux produits répondant à la formule générale:

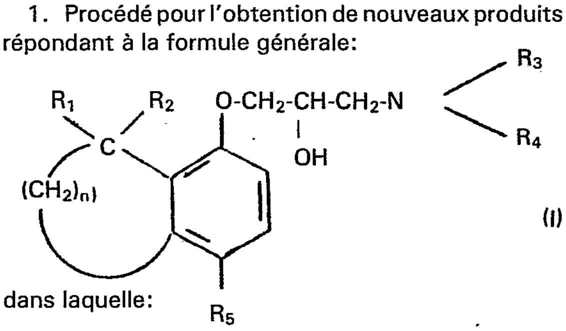

(I)

dans laquelle:

— $R_1$ et $R_2$ pris ensemble représentent un atome d'oxygène = O; ou encore $R_1$ représente l'hydrogène et $R_2$ représente un radical OH,
— $R_3$ désigne un atome d'hydrogène ou un radical alkyle droit ou ramifié ayant de 1 à 6 atomes de carbone,
— $R_4$ représente un radical alkyle droit ou ramifié ayant de 1 à 6 atomes de carbone ou un groupe alkynyle ayant de 2 à 6 atomes de C,
— $R_5$ désigne un atome d'hydrogène, un groupe alkyle ayant de 1 à 4 atomes de carbone ou un groupe acylamine,
— n = 3 ou 4,
ainsi que celle des sels d'addition d'acides desdits produits, caractérisé en ce qu'il consiste à faire réagir sur une cétone de formule:

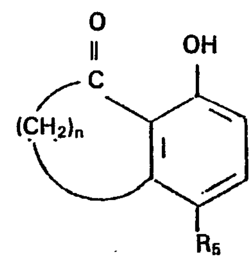

de l'épichlorhydrine en présence d'un agent alcalin au sein d'un solvant tel que de l'eau, de l'éthanol, un polyalcool ou un éther de polyol et à faire réagir le produit obtenu sur une amine de formule $NH \langle {R_3 \atop R_4}$, après quoi on obtient les dérivés dans lesquels $R_1$ est H et $R_2$ est OH par réduction à l'aide d'un borohydrure de sodium.

2. Procédé selon la revendication 1, caractérisé en ce que les dérivés optiquement actifs sont obtenus par traitement du racémique correspondant à l'aide d'un acide optiquement actif.

**Patentansprüche für die Vertragsstaaten:**
BE, CH, DE, FR, GB, IT, LI, LU, NL, SE

1. Neue Produkte, dadurch gekennzeichnet, dass sie der allgemeinen Formel

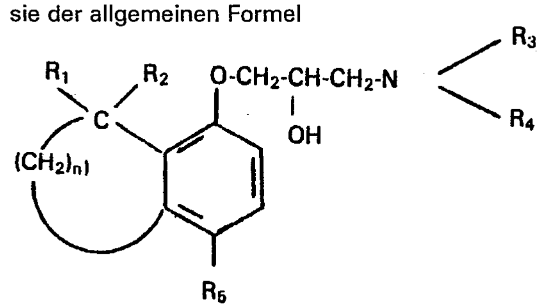

(I)

entsprechen, worin
— $R_1$ und $R_2$ zusammen ein Sauerstoffatom = O darstellen, oder aber $R_1$ Wasserstoff und $R_2$ eine OH-Gruppe bedeutet,
— $R_3$ ein Wasserstoffatom oder eine gerade oder verzweigte Alkylgruppe mit 1 bis 6 Kohlenstoffatomen bezeichnet,
— $R_4$ für eine gerade oder verzweigte Alkylgruppe mit 1 bis 6 Kohlenstoffatomen oder eine Alkinylgruppe mit 2 bis 6 Kohlenstoffatomen steht,

— $R_5$ ein Wasserstoffatom, eine Alkylgruppe mit 1 bis 4 Kohlenstoffatomen oder eine Acylamingruppe bedeutet,
— n = 3 oder 4,
sowie die Säureadditionssalze dieser Produkte.

2. Neue Produkte nach Anspruch 1, dadurch gekennzeichnet, dass sie in racemischer Form oder in Form eines ihrer optischen Isomeren vorliegen.

3. Verfahren zur Herstellung der Produkte nach Anspruch 1, dadurch gekennzeichnet, dass man ein Keton der Formel

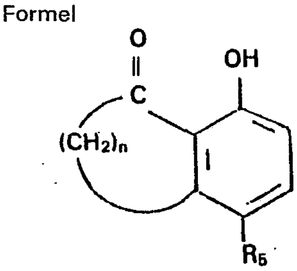

mit Epichlorhydrin in Gegenwart eines alkalischen Mittels in einem Lösungsmittel, wie Wasser, Äthanol, einem Polyalkohol oder einem Polyoläther zur Umsetzung bringt und das erhaltene Produkt mit einem Amin der Formel $HN \lessdot {}^{R_3}_{R_4}$ umsetzt, worauf man durch Reduktion mittels Natriumborhydrid die Derivate erhält, in denen $R_1$ H und $R_2$ OH darstellt.

4. Verfahren nach Anspruch 3, dadurch gekennzeichnet, dass die optisch aktiven Derivate durch Behandeln des entsprechenden Racemats mit einer optisch aktiven Säure erhalten werden.

5. Medikament mit Wirkung insbesondere auf das kardiovaskuläre System, dadurch gekennzeichnet, dass es als aktive Substanz ein Produkt nach einem der Ansprüche 1 und 2 enthält.

6. Medikament nach Anspruch 5, dadurch gekennzeichnet, dass es zur Verabreichung in oraler Form, in Form von Suppositorien oder in injizierbarer Form in einer Menge von 1 bis 10 mg Wirkstoff pro Tag formuliert ist.

**Patentansprüche für den Vertragsstaat:**
AT

1. Verfahren zur Herstellung neuer Produkte der allgemeinen Formel

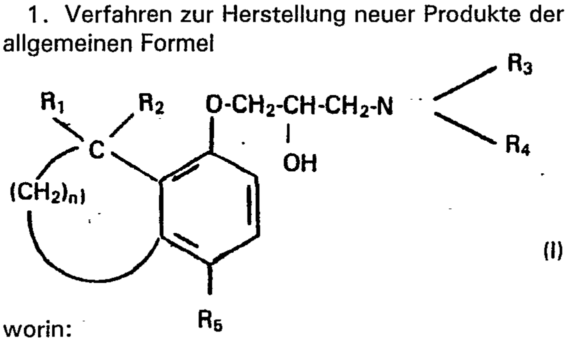

(I)

worin:
— $R_1$ und $R_2$ zusammen ein Sauerstoffatom = O darstellen, oder aber $R_1$ Wasserstoff und $R_2$ eine OH-Gruppe bedeutet,
— $R_3$ ein Wasserstoffatom oder eine gerade oder verzweigte Alkylgruppe mit 1 bis 6 Kohlenstoffatomen bezeichnet,
— $R_4$ für eine gerade oder verzweigte Alkylgruppe mit 1 bis 6 Kohlenstoffatomen oder eine Alkinylgruppe mit 2 bis 6 Kohlenstoffatomen steht,
— $R_5$ ein Wasserstoffatom, eine Alkylgruppe mit 1 bis 4 Kohlenstoffatomen oder eine Acylamingruppe bedeutet,
— n = 3 oder 4,
sowie der Säureadditionssalze dieser Produkte, dadurch gekennzeichnet, dass man ein Keton der Formel

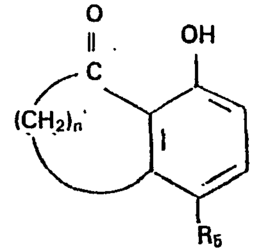

mit Epichlorhydrin in Gegenwart eines alkalischen Mittels in einem Lösungsmittel, wie Wasser, Äthanol, einem Polyalkohol oder einem Polyoläther zur Umsetzung bringt und das erhaltene Produkt mit einem Amin der Formel $HN \lessdot {}^{R_3}_{R_4}$ umsetzt, worauf man durch Reduktion mittels Natriumborhydrid die Derivate erhält, in denen $R_1$ H und $R_2$ OH darstellt.

2. Verfahren nach Anspruch 1, dadurch gekennzeichnet, dass die optisch aktiven Derivate durch Behandeln des entsprechenden Racemats mit einer optisch aktiven Säure erhalten werden.

**Claims for the Contracting States:**
BE, CH, DE, FR, GB, IT, LI, LU, NL, SE

1. Novel products, characterized in that they respond to general formula:

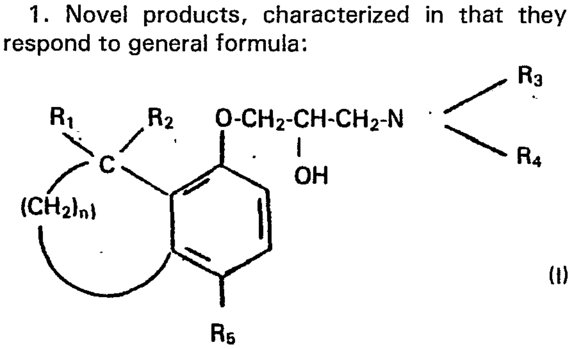

(I)

in which:
— $R_1$ and $R_2$ taken together represent an atom of oxygen = O or else $R_1$ represents hydrogen and $R_2$ represents an OH radical,
— $R_3$ designates an atom of hydrogen or a straight or branched alkyl radical having from 1 to 6 atoms of carbon,
— $R_4$ represents a straight or branched alkyl radical having from 1 to 6 atoms of carbon, or an alkynyl group having 2 to 6 atoms of carbon,
— $R_5$ designates an atom of hydrogen, an alkyl group having from 1 to 4 atoms of carbon or an acylamine group,
— n = 3 or 4;
as well as the acid addition salts of said products.

2. The novel products according to claim 1, characterized in that they are in racemic form or in the form of one of their optical isomers.

3. Process for preparing the products according to claim 1, characterized in that it comprises the steps of reacting on a ketone of formula:

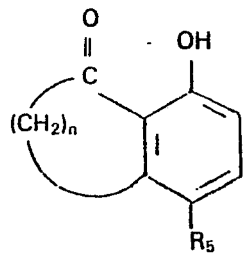

epichlorohydrin in the presence of an alkaline agent within a solvent such as water, ethanol, a polyalcohol or an ether of polyol and of reacting the product obtained on an amine of formula $HN<^{R_3}_{R_4}$ after which the derivatives in which $R_1$ is H and $R_2$ is OH are obtained by reduction with the aid of a sodium borohydride.

4. The process according to claim 3, characterized in that the optically active derivatives are obtained by treatment of the corresponding racemic with the aid of an optically active acid.

5. Drug particularly active on the cardiovascular system, characterized in that it contains, as active substance, the product according to any one of claims 1 and 2.

6. The drug according to claim 5, characterized in that it is prepared for administration in oral form, in the form of suppositories or in injectable form at a rate of 1 to 10 mg of active ingredient per day.

**Claims for the Contracting State:**
AT

1. Process for obtaining novel products, responding to general formula:

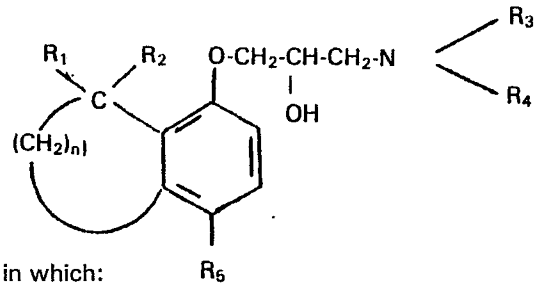

(I)

in which:

— $R_1$ and $R_2$ taken together represent an atom of oxygen = O or else $R_1$ represents hydrogen and $R_2$ represents an OH radical,

— $R_3$ designates an atom of hydrogen or a straight or branched alkyl radical having from 1 to 6 atoms of carbon,

— $R_4$ represents a straight or branched alkyl radical having from 1 to 6 atoms of carbon, or an alkynyl group having 2 to 6 atoms of carbon,

— $R_5$ designates an atom of hydrogen, an alkyl group having from 1 to 4 atoms of carbon or an acylamine group,

— $n = 3$ or 4;

as well as the acid addition salts of said products, characterized in that it consists in reacting on a ketone of formula:

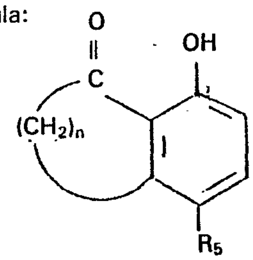

epichlorohydrin in the presence of an alkaline agent within a solvent such as water, ethanol, a polyalcohol or an ether of polyol and of reacting the product obtained on an amine of formula $HN<^{R_3}_{R_4}$ after which the derivatives in which $R_1$ is H and $R_2$ is OH are obtained by reduction with the aid of a sodium borohydride.

2. The process according to claim 1, characterized in that the optically active derivatives are obtained by treatment of the corresponding racemic with the aid of an optically active acid.